# EUROPEAN PATENT APPLICATION

(11) **EP 3 610 800 A1**
(43) Date of publication of application: **19.02.2020**
(21) Application number: 18785075.5
(22) Date of filing: 10.04.2018
(51) Int. Cl.: A61B 17/06, D02J 3/02, A61B 17/00, B29C 45/00

(54) **ADHESIVE BARBED SUTURE AND METHOD FOR PRODUCING SAME**

(30) Priority: 11.04.2017 KR 20170046552; 06.04.2018 KR 20180040421
(71) Applicant: Yu, Won-Seok, Daejeon 34817 (KR)
(72) Inventor: Yu, Won-Seok, Daejeon 34817 (KR)
(74) Representative: Isarpatent
(86) International application number: PCT/KR2018/004216
(87) International publication number: WO 2018/190625

(57) **Abstract**

The present invention relates to an adhesive barbed suture which is a barbed suture having barbs and barb grooves formed on the outer peripheral surface of a suture body. The tail end of the barb and the tail end of the barb groove, which are in contact with the outer peripheral surface of the suture body, are integrally bonded to the suture body at the same position on the outer peripheral surface of the suture body. The front end of the barb is spaced apart from the front end of the barb groove by being upwardly inclined at a certain angle on the outer peripheral surface of the suture body, and the barbs and the barb grooves are formed at regular intervals along a spiral path of the suture body. The front end of the barb is aligned in the longitudinal direction toward the axial direction of the suture body, and the front end of the barb groove is aligned in the longitudinal direction toward the spiral direction of the suture body while having a spiral angle with respect to the front end of the barb. Thus, the adhesive force of the barbed suture is maximized by means of combined biaxial stress in which stress by an external force in the axial direction applied to the front end of the barb and stress by an external force in the spiral direction applied to the front end of the barb groove are simultaneously applied.

## Description

### [Technical Field]

The present invention relates to an adhesive barbed suture for connecting or fixing animal tissues under various medical environments, and more particularly to an adhesive barbed suture which may maximize adhesive force thereof after insertion into animal tissue when suturing and thus promote effectiveness and safety depending on application of the suture, and a method for producing the same.

### [Background Art]

In general, sutures are used to connect or suture various damaged regions of animal tissues, such as skin, muscles, tendons, internal organs, bony tissues, nerves, blood vessels, etc., and incision regions caused by a surgical operation.

These sutures are divided into a non-absorbable suture formed of a material, such as silk, cotton, nylon, Dafilon, polyester, polypropylene or stainless steel, and a bioabsorbable suture formed of a material, such as a lactic acid (L-lactide) polymer, a glycolic acid (glycolide) polymer, a copolymer of L-lactide and glycolide, copolymers of these polymers and copolymers and caprolactone or trimethylene carbonate, or polydi-oxanone, chitosan or derivatives thereof. Particularly, the bioabsorbable suture does not need to be removed from a patient, thus providing convenience in use thereof.

As a conventional suture is used to stitch incised tissue, a usage process for forming at least one knot by a user is required. However, since various knot methods for knotting the suture are present, these methods are very complicated and thus require the user, i.e., a surgeon, to be highly trained and a considerably long time is taken to knot the suture during surgery, there is a limit to promote minimization of a time during which an operation is performed and a time during which a patient's lesion is open during operations requiring a large quantity of sutures and knots, such as heart surgery.

Further, knots may be damaged, loosened or untied, and thus, it is known that sutures may be destroyed and cosmetically leave external scars. Therefore, the need for development of technology about a suture which may be used without formation of knots arises.

In order to solve these problems, barbed sutures have been commercialized via many processes of development after initiation of ideas thereof, and these barbed sutures compensates for the above-described problems caused by the conventional sutures requiring knots and thus provide various advantages. The barbed sutures have one or more protruding barbs formed at regular intervals on a suture body in the longitudinal direction of the suture body, and have unidirectional mobility due to the structure and function of the barbs.

That is, due to the structure of the barbs, movement of which in one direction may be suppressed, after the barbed suture is inserted into tissue in one direction, movement of the barbed suture in the opposite direction is suppressed. Accordingly, due to these properties, the barbed suture may be used without knots, in contrast with the conventional sutures.

The basic idea and design of a barbed suture having industrial applicability is disclosed in U.S. Patent No. 3,123,077 to J. H. Alcamo in the mid-1950s. The corresponding conventional technology describes a cord for sewing human tissue which has unidirectional mobility (i.e., which may pass through the tissue in one direction, but suppress movement in the opposite direction), and such a property corresponds to the basic attribution of barbed sutures. As a unit for achieving this property, an elongate cord including a body portion and sharp-edged resilient barbs protruding from the body portion at an acute angle is described. However, since this structure removes the need for knots, but has no anchor part and may thus cause sliding in one movable direction, the structure has a limit to formation of a conventional loop or simplification of a suturing method for forming sinusoidal-type sutures.

Further, U.S. Patent No. 5,053,047 discloses a bioabsorbable barbed suture including a spherical anchor part at one end thereof. This structure may provide an advantage in that suturing may be performed merely by inserting the suture without formation of a loop or sinusoidal-type sutures, like in a continuous suturing method. However, although a suture structure, in which one end of the suture may perform an anchoring function due to the anchor part, is provided, the bioabsorbable barbed suture has a drawback in which a part of the anchored suture must be exposed to the outside. Such a drawback causes poor appearance quality, and may thus increase the possibility of side effects, such as difficulty in management of a sutured part, damage to the sutured part, bleeding, infection, inflammation, etc.

In addition, U.S. Patent No. 5,374,268 discloses a surgical device including anchoring members formed of a bioabsorbable material and arranged in both directions so that barbs protruding from both front ends of a suture are opposite each other. Further, U.S. Patent No. 5,931,855 discloses a barbed suture having bidirectional arrangement in which barbs located at a part corresponding to about half the length of the barbed suture are opposite each other. Korean Patent Registration No. 10-0545105 (published as PCT Patent Publication No. WO 2005/096956) discloses a barbed suture having bidirectional arrangement, including a clear section having no barbs at a central region thereof and barbs formed at both sides of the clear section so as to be opposite each other with the clear section therebetween.

In the structures disclosed in U.S. Patent No. 5,374,268, U.S. Patent No. 5,931,855, and Korean Patent Registration No. 10-0545105 (published as PCT Patent Publication No. WO 2005/096956), both front ends of each structure may be inserted into different points of a diseased area and thereby suturing is possible through the conventional suturing method or a simple suturing method, and, as disclosed in Korean Patent Registration No. 10-0545105, the central portion of the suture may perform an anchoring function at one sutured part in some surgical operation methods. These characteristics may serve as effective advantages in plastic surgery, such as tissue lifting. However, when the diseased area is sutured, a wide sutured part and a wide additionally secondary diseased area may be caused due to the structure of the suture and the suturing method, and in plastic surgery, such as tissue lifting, two different needles pass through tissue and a wide diseased area is additionally caused by the surgery.

Further, in the barbed sutures, barbs are formed by forming protrusions on a body of the barbed suture or cutting the body of the barbed suture, and these barbs satisfy the basic design and structure of barbs which are inserted into tissue in one direction and then restrain movement in the opposite direction. However, these barbed sutures still retain sliding properties in one direction after insertion into the tissue due to the basic properties of the barbed sutures, and these properties frequently cause side effects, such as protrusions or dimples according to the displacement of the sutures. Therefore, an adhesive suture which has resistance to movement in both directions after insertion into tissue has been required as an ideal suture.

Therefore, PCT Patent Publication No. WO 2006/061868 discloses a fixed barbed suture in which barb segments formed in both directions are arranged so as to be opposite each other. Such a structure is effective as a fixed barbed suture having resistance to movement in both directions after insertion of the suture, but when the suture is inserted into tissue, the suture may not be smoothly inserted and thereby has a suturing performance problem at an accurate suturing point of a sutured part.

Further, Korean Patent Registration No. 10-1043179 discloses a suture for surgery including a core wire and an outside wire for anti-sliding which is spirally wound on the core wire in the longitudinal direction of the core wire. In addition, Korean Patent Registration No. 10-1237481 discloses a medical suture in which a continuous spiral projection is formed on one projection section along the outer peripheral surface of a suture body, and the other projection section is an outward flange. The sutures disclosed in the two patent documents have a structure in which the suture may be smoothly inserted into tissue and fibrous tissue may be grown and engrafted into the spiral uneven part formed on the suture due to a regeneration process of the tissue after insertion of the tissue, thus being capable of providing firmer tissue fixing effects. However, these sutures have no fixing elements, such as sharp barbs for physically fixing dense tissue, immediately after insertion of the sutures into the tissue and thus have poor tissue fixing effects which is the intended purpose, before the fixing effects caused by growth and engraftment of the fibrous tissue.

Particularly, if the above-described sutures having projections, including the conventional barbed sutures, are applied to tissue lift surgery, such as face lift surgery, which specially requires fixing force of a suture, lifting effects are poor due to absence of barbs suitable for tissue lifting, or although barbs for tissue lifting are present and thus tissue lifting is possible, coupling between the suture and lifted tissue and surrounding tissues at a lifted position is weak and thus, as time goes on, the inserted suture is slid in the reverse direction to the lifting direction due to gravity, muscular movement, etc. and the lifted tissue is again lowered to the initial position thereof. This problem may be a theoretical problem of the conventional barbed sutures, i.e., the sutures having unidirectional mobility. In order to compensate for this problem, surgery is performed through a more complicated method, such as anchoring of the end or one part of the suture to tissue, and thereby, it is obvious that the risk of side effects, such as scarring, infection, inflammation, etc., may be increased.

In order to improve performance of the sutures against the above side effects, inventions and efforts to form barbs in all 360 degree directions based on a suture body have been attempted, and these attempts are intended to increase fixing force of the barbs, to uniformly distribute load applied to the barbed suture, and thus, to improve durability of the suture due to increase in fixing force of the suture and uniform distribution of the load and to prevent formation of dimples caused by damage to the barbs at a specific region. However, the barbed suture in which the barbs are formed in all 360 degree directions based on the suture body may cause negative problems in addition to these positive effects. That is, omnidirectional barb damage in 360 degree orientations based on the suture body may be caused, and in this case, the outer diameter of the suture body which maintains the strength of the suture is excessively reduced, thus extremely lowering the properties of the barbed suture.

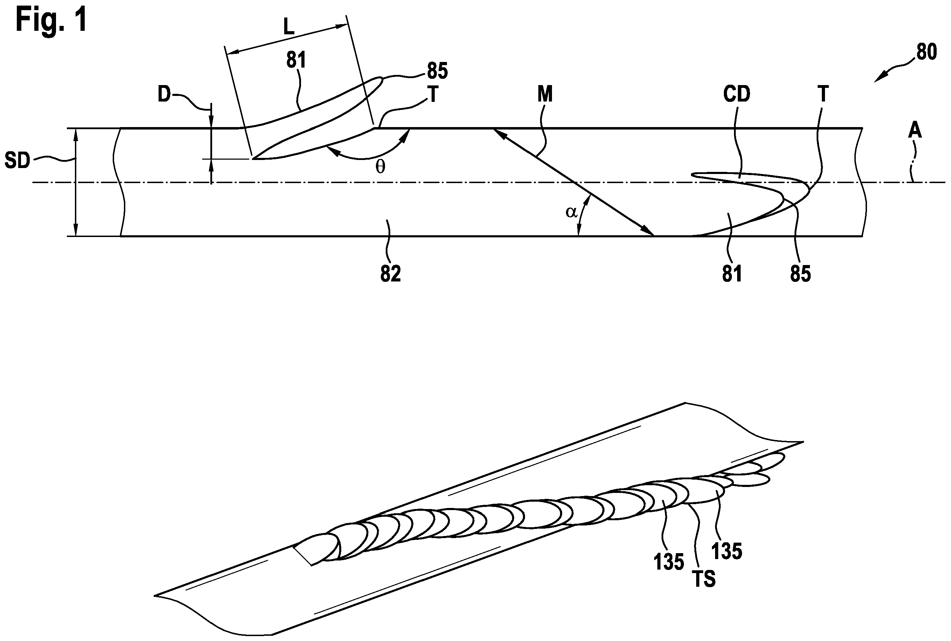

In order to solve all the above-described conventional problems, Korean Patent Registration No. 10-1172829 discloses a barbed suture having barbs formed in a spiral direction on the outer peripheral surface of the suture, the inventor of the present invention filed a patent application for an adhesive barbed suture having barbs formed in a spiral direction on the outer peripheral surface of the suture, on May 17, 2016, and, as shown in FIG. 1, in this barbed suture having the barbs formed in the spiral direction, the suture may be slid due to external force in the opposite direction like the conventional barbed suture, or be slid and thus separated from tissue due to external force in the spiral direction.

### [Disclosure]

### [Technical Problem]

Therefore, the present invention has been made in view of the above problems, and it is an object of the present invention to provide an adhesive barbed suture having barbs and barb grooves formed on an outer peripheral surface of a suture body and a method for producing the same, in which a tail end of the barb and a tail end of the barb groove, which are in contact with the outer peripheral surface of the suture body, are integrally bonded to the suture body at the same position on the outer peripheral surface of the suture body, a front end of the barb is spaced apart from a front end of the barb groove by being upwardly inclined at a certain angle on the outer peripheral surface of the suture body, the barbs and the barb grooves are formed at regular intervals along a spiral path of the suture body, and the front end of the barb is aligned in a longitudinal direction toward an axial direction of the suture body and the front end of the barb groove is aligned in the longitudinal direction toward a spiral direction of the suture body while having a spiral angle with respect to the front end of the barb so that adhesive force of the barbed suture is increased by means of combined biaxial stress in which stress by external force in the axial direction applied to the front end of the barb and stress by external force in the spiral direction applied to the front end of the barb groove are simultaneously applied.

### [Technical Solution]

In accordance with an aspect of the present invention, the above and other objects can be accomplished by the provision of an adhesive barbed suture having barbs and barb grooves formed on an outer peripheral surface of a suture body, wherein a tail end of the barb and a tail end of the barb groove, which are in contact with the outer peripheral surface of the suture body, are integrally bonded to the suture body at the same position on the outer peripheral surface of the suture body, a front end of the barb is spaced apart from a front end of the barb groove by being upwardly inclined at a certain angle on the outer peripheral surface of the suture body, the barbs and the barb grooves are formed at regular intervals along a spiral path of the suture body, and the front end of the barb is aligned in a longitudinal direction toward an axial direction of the suture body and the front end of the barb groove is aligned in the longitudinal direction toward a spiral direction of the suture body while having a spiral angle with respect to the front end of the barb so that adhesive force of the barbed suture is increased by means of combined biaxial stress in which stress by external force in the axial direction applied to the front end of the barb and stress by external force in the spiral direction applied to the front end of the barb groove are simultaneously applied.

The spiral angle of the front end of the barb groove with respect to the front end of the barb may be within a range of 3-80 degrees.

When the barbs and the barb grooves are formed at the regular intervals along the spiral path of the suture body, the interval may be less than 3 times a diameter of the suture body.

The front end of the barb and the front end of the barb groove may be formed with a regular thickness so as to have a designated angle.

One protruding barb or two or more protruding barbs, selected from the group consisting of a wedge-type protruding barb, a right-angled triangular protruding barb, a wedge-type conical protruding barb, a right-angled conical protruding barb and a knot-type protruding barb, may be formed on the outer peripheral surface of the suture body within the longitudinal interval between the barb and the barb.

In accordance with another aspect of the present invention, there is provided a method for producing the adhesive barbed suture, wherein the adhesive barbed suture is produced so as to have a designated length through injection molding by injecting a liquid resin into a mold cavity having a shape of the suture body configured such that the barb grooves having the tail ends and the front ends and the barbs having the tail ends and the front ends to have a shape corresponding to the barb grooves having the tail ends and the front ends are formed at the regular intervals on the outer peripheral surface of the suture body, the tail end of the barb and the tail end of the barb groove, which are in contact with the outer peripheral surface of the suture body, are integrally bonded to the suture body at the same position on the outer peripheral surface of the suture body, the front end of the barb is spaced apart from the front end of the barb groove by being upwardly inclined at the certain angle on the outer peripheral surface of the suture body, the barbs and the barb grooves are formed at the regular intervals along the spiral path of the suture body, the front end of the barb is aligned in the longitudinal direction toward the axial direction of the suture body, and the front end of the barb groove is aligned in the longitudinal direction toward the spiral direction of the suture body while having the spiral angle with respect to the front end of the barb.

In accordance with another aspect of the present invention, there is provided a method for producing the adhesive barbed suture, wherein the adhesive barbed suture is produced by forming the barbs having the tail ends and the front ends to have a shape corresponding to the barb grooves having the tail ends and the front ends by forming the barb grooves having the tail ends and the front ends at the regular intervals on the outer peripheral surface of the suture body so that the tail end of the barb and the tail end of the barb groove, which are in contact with the outer peripheral surface of the suture body, are integrally bonded to the suture body at the same position on the outer peripheral surface of the suture body, the front end of the barb is spaced apart from the front end of the barb groove by being upwardly inclined at the certain angle on the outer peripheral surface of the suture body, through cutting using a blade cutting machine or a laser cutting machine, and then by twisting the suture body by axially rotating the suture body so that the barbs and the barb grooves are formed at the regular intervals along the spiral path of the suture body, the front end of the barb is aligned in the longitudinal direction toward the axial direction of the suture body, and the front end of the barb groove is aligned in the longitudinal direction toward the spiral direction of the suture body while having the spiral angle with respect to the front end of the barb.

In accordance with another aspect of the present invention, there is provided a method for producing the adhesive barbed suture, wherein the adhesive barbed suture is produced by forming the barb grooves having the tail ends and the front ends and the barbs having the tail ends and the front ends to have a shape corresponding to the barb grooves having the tail ends and the front ends at the regular intervals on the outer peripheral surface of the suture body so that the tail end of the barb and the tail end of the barb groove, which are in contact with the outer peripheral surface of the suture body, are integrally bonded to the suture body at the same position on the outer peripheral surface of the suture body, the front end of the barb is spaced apart from the front end of the barb groove by being upwardly inclined at the certain angle on the outer peripheral surface of the suture body, through compression molding using a compression molding machine, and then by twisting the suture body by axially rotating the suture body so that the barbs and the barb grooves are formed at the regular intervals along the spiral path of the suture body, the front end of the barb is aligned in the longitudinal direction toward the axial direction of the suture body, and the front end of the barb groove is aligned in the longitudinal direction toward the spiral direction of the suture body while having the spiral angle with respect to the front end of the barb.

In accordance with yet another aspect of the present invention, there is provided a method for producing the adhesive barbed suture, wherein the adhesive barbed suture is produced by forming the barb grooves having the tail ends and the front ends and the barbs having the tail ends and the front ends to have a shape corresponding to the barb grooves having the tail ends and the front ends at the regular intervals on the outer peripheral surface of the suture body so that the tail end of the barb and the tail end of the barb groove, which are in contact with the outer peripheral surface of the suture body, are integrally bonded to the suture body at the same position on the outer peripheral surface of the suture body, the front end of the barb is spaced apart from the front end of the barb groove by being upwardly inclined at the certain angle on the outer peripheral surface of the suture body, through compression molding using a compression molding machine, and then by performing compression molding while rotating the suture body so that the barbs and the barb grooves are formed at the regular intervals along the spiral path of the suture body, the front end of the barb is aligned in the longitudinal direction toward the axial direction of the suture body, and the front end of the barb groove is aligned in the longitudinal direction toward the spiral direction of the suture body while having the spiral angle with respect to the front end of the barb.

### [Advantageous effects]

In an adhesive barbed suture and a method for producing the same in accordance with the present invention, a tail end of a barb and a tail end of a barb groove, which are in contact with the outer peripheral surface of a suture body, are integrally bonded to the suture body at the same position on the outer peripheral surface of the suture body, a front end of the barb is spaced apart from a front end of the barb groove by being upwardly inclined at a certain angle on the outer peripheral surface of the suture body, the barbs and the barb grooves are formed at regular intervals along a spiral path of the suture body, and the front end of the barb is aligned in a longitudinal direction toward an axial direction of the suture body and the front end of the barb groove is aligned in the longitudinal direction toward a spiral direction of the suture body while having a spiral angle with respect to the front end of the barb, so that the adhesive force of the barbed suture is increased by means of combined biaxial stress in which stress by external force in the axial direction applied to the front end of the barb and stress by external force in the spiral direction applied to the front end of the barb groove are simultaneously applied, and thus knots are not necessary when using the suture, and the suture may be rapidly and firmly fixed by maximizing fixing force between tissues and surrounding tissues, and the suture.

Moreover, in the adhesive barbed suture and the method for producing the same in accordance with the present invention, torsional moment may be applied to tissues surrounding a sutured part by external force in a tensile direction occurring due to movement of the suture with respect to a region into which the suture is inserted or a sutured area, and thereby, suturing may be firmly enabled due to strong adhesive effects against the movement of the suture with respect to the inserted area or the sutured area.

Further, in the adhesive barbed suture and the method for producing the same in accordance with the present invention, strong adhesiveness between the suture, and sutured tissue and surrounding tissues may lower the possibility of serious side effects, such as rupture of the sutured area, and promote suturing performance having excellent safety.

In addition, in the adhesive barbed suture and the method for producing the same in accordance with the present invention, damage to the suture body occurring due to tearing of the barbs may be minimized, and thus, maintenance of durability of the suture after use may be maximized.

Also, in the adhesive barbed suture and the method for producing the same in accordance with the present invention, a production process of the adhesive barbed suture may be simplified, and simultaneously, the produced adhesive barbed suture may have excellent commercial utility.

### [Description of Drawings]

FIG. 1 is a perspective view illustrating the structure of a conventional barbed suture having a spiral structure.
FIG. 2 is a partial perspective view illustrating the structure of an adhesive barbed suture in accordance with the present invention.
FIG. 3 is a longitudinal-sectional view illustrating the structure of the adhesive barbed suture in accordance with the present invention.
FIG. 4 is an overall perspective view illustrating the structure of the adhesive barbed suture in accordance with the present invention.
FIG. 5 is a conceptual view illustrating application of bidirectional stress to the adhesive barbed suture in accordance with the present invention.
FIG. 6 is a schematic plan view of the adhesive barbed suture in accordance with the present invention.
FIG. 7 is a view illustrating a real product of the adhesive barbed suture in accordance with the present invention.
FIG. 8 is a view illustrating the configuration of wedge-type protruding barbs of the adhesive barbed suture in accordance with the present invention.
FIG. 9 is a view illustrating the configuration of right-angled triangular protruding barbs of the adhesive barbed suture in accordance with the present invention.
FIG. 10 is a view illustrating the configuration of wedge-type conical protruding barbs of the adhesive barbed suture in accordance with the present invention.
FIG. 11 is a view illustrating the configuration of right-angled conical protruding barbs of the adhesive barbed suture in accordance with the present invention.
FIG. 12 is a view illustrating the configuration of knot-type protruding barbs of the adhesive barbed suture in accordance with the present invention.

### [Best Mode]

The present invention provides an adhesive barbed suture having barbs and barb grooves formed on an outer peripheral surface of a suture body, wherein a tail end of the barb and a tail end of the barb groove, which are in contact with the outer peripheral surface of the suture body, are integrally bonded to the suture body at the same position on the outer peripheral surface of the suture body, a front end of the barb is spaced apart from a front end of the barb groove by being upwardly inclined at a certain angle on the outer peripheral surface of the suture body, the barbs and the barb grooves are formed at regular intervals along a spiral path of the suture body, and the front end of the barb is aligned in a longitudinal direction toward an axial direction of the suture body and the front end of the barb groove is aligned in the longitudinal direction toward a spiral direction of the suture body while having a spiral angle with respect to the front end of the barb so that adhesive force of the barbed suture is increased by means of combined biaxial stress in which stress by external force in the axial direction applied to the front end of the barb and stress by external force in the spiral direction applied to the front end of the barb groove are simultaneously applied.

The spiral angle of the front end of the barb groove with respect to the front end of the barb is within a range of 3-80 degrees.

When the barbs and the barb grooves are formed at the regular intervals along the spiral path of the suture body, the interval is less than 3 times a diameter of the suture body.

The front end of the barb and the front end of the barb groove are formed with a regular thickness so as to have a designated angle.

One protruding barb or two or more protruding barbs, selected from the group consisting of a wedge-type protruding barb, a right-angled triangular protruding barb, a wedge-type conical protruding barb, a right-angled conical protruding barb and a knot-type protruding barb, are formed on the outer peripheral surface of the suture body within the longitudinal interval between the barb and the barb.

Further, the present invention provides a method for producing the adhesive barbed suture, wherein the adhesive barbed suture is produced so as to have a designated length through injection molding by injecting a liquid resin into a mold cavity having a shape of the suture body configured such that the barb grooves having the tail ends and the front ends and the barbs having the tail ends and the front ends to have a shape corresponding to the barb grooves having the tail ends and the front ends are formed at the regular intervals on the outer peripheral surface of the suture body, the tail end of the barb and the tail end of the barb groove, which are in contact with the outer peripheral surface of the suture body, are integrally bonded to the suture body at the same position on the outer peripheral surface of the suture body, the front end of the barb is spaced apart from the front end of the barb groove by being upwardly inclined at the certain angle on the outer peripheral surface of the suture body, the barbs and the barb grooves are formed at the regular intervals along the spiral path of the suture body, the front end of the barb is aligned in the longitudinal direction toward the axial direction of the suture body, and the front end of the barb groove is aligned in the longitudinal direction toward the spiral direction of the suture body while having the spiral angle with respect to the front end of the barb.

Further, the present invention provides a method for producing the adhesive barbed suture, wherein the adhesive barbed suture is produced by forming the barbs having the tail ends and the front ends to have a shape corresponding to the barb grooves having the tail ends and the front ends by forming the barb grooves having the tail ends and the front ends at the regular intervals on the outer peripheral surface of the suture body so that the tail end of the barb and the tail end of the barb groove, which are in contact with the outer peripheral surface of the suture body, are integrally bonded to the suture body at the same position on the outer peripheral surface of the suture body, the front end of the barb is spaced apart from the front end of the barb groove by being upwardly inclined at the certain angle on the outer peripheral surface of the suture body, through cutting using a blade cutting machine or a laser cutting machine, and then by twisting the suture body by axially rotating the suture body so that the barbs and the barb grooves are formed at the regular intervals along the spiral path of the suture body, the front end of the barb is aligned in the longitudinal direction toward the axial direction of the suture body, and the front end of the barb groove is aligned in the longitudinal direction toward the spiral direction of the suture body while having the spiral angle with respect to the front end of the barb.

Further, the present invention provides a method for producing the adhesive barbed suture, wherein the adhesive barbed suture is produced by forming the barb grooves having the tail ends and the front ends and the barbs having the tail ends and the front ends to have a shape corresponding to the barb grooves having the tail ends and the front ends at the regular intervals on the outer peripheral surface of the suture body so that the tail end of the barb and the tail end of the barb groove, which are in contact with the outer peripheral surface of the suture body, are integrally bonded to the suture body at the same position on the outer peripheral surface of the suture body, the front end of the barb is spaced apart from the front end of the barb groove by being upwardly inclined at the certain angle on the outer peripheral surface of the suture body, through compression molding using a compression molding machine, and then by twisting the suture body by axially rotating the suture body so that the barbs and the barb grooves are formed at the regular intervals along the spiral path of the suture body, the front end of the barb is aligned in the longitudinal direction toward the axial direction of the suture body, and the front end of the barb groove is aligned in the longitudinal direction toward the spiral direction of the suture body while having the spiral angle with respect to the front end of the barb.

The present invention provides a method for producing the adhesive barbed suture, wherein the adhesive barbed suture is produced by forming the barb grooves having the tail ends and the front ends and the barbs having the tail ends and the front ends to have a shape corresponding to the barb grooves having the tail ends and the front ends at the regular intervals on the outer peripheral surface of the suture body so that the tail end of the barb and the tail end of the barb groove, which are in contact with the outer peripheral surface of the suture body, are integrally bonded to the suture body at the same position on the outer peripheral surface of the suture body, the front end of the barb is spaced apart from the front end of the barb groove by being upwardly inclined at the certain angle on the outer peripheral surface of the suture body, through compression molding using a compression molding machine, and then by performing compression molding while rotating the suture body so that the barbs and the barb grooves are formed at the regular intervals along the spiral path of the suture body, the front end of the barb is aligned in the longitudinal direction toward the axial direction of the suture body, and the front end of the barb groove is aligned in the longitudinal direction toward the spiral direction of the suture body while having the spiral angle with respect to the front end of the barb.

Hereinafter reference will now be made in detail to various embodiments of the present invention, examples of which are illustrated in the accompanying drawings and described below. While the invention will be described in conjunction with exemplary embodiments, it will be understood that present description is not intended to limit the invention to those exemplary embodiments and drawings.

First, an adhesive barbed suture in accordance with the present invention includes a suture body 10, barb grooves 30 and barbs 20, and the barbs 20 and the barb grooves 30 respectively have tail ends 31 and 21 and front ends 22 and 32 which correspond to each other.

The adhesive barbed suture is formed of a material having flexibility and strength which are proper to connect or suture various damaged regions of animal tissues, such as skin, muscles, tendons, internal organs, bony tissues, nerves, blood vessels, etc., and incision resions caused by a surgical operation, rigidity which is sufficient to prevent shape deformation or cutting of the adhesive barbed suture, and biocompatibility.

For example, the adhesive barbed suture may most preferably be formed of a bioabsorbable polymer material which may be absorbed by tissues after a certain period of time, and a lactic acid (L-lactide) polymer, a glycolic acid (glycolide) polymer, a caprolactone polymer, a trimethylene carbonate polymer, a dioxanone polymer, chitosan and derivatives thereof may be used independently or copolymers therebetween or blending materials thereof may be used as the bioabsorbable polymer material, and silk, cotton, nylon, Dafilon, polyester, polypropylene, stainless steel, etc. may be used as a non-absorbable material, without being limited thereto.

The reason why the bioabsorbable polymer material is more proper is that, in contrast with the non-absorbable material, even if the barb grooves 30 and the barbs 20 are ripped to or separated into fine pieces and thus are present in a human body, when the adhesive barbed suture in accordance with the present invention is used, they may be absorbed in the human body without side effects, and, in the case of the non-absorbable material, the fine suture pieces may continuously stimulate nerves in the human body and thus cause side effects, such as pain, etc.

The suture body 10 has a circular cross-section and extends in the longitudinal direction, a surgical needle (not shown) which may be inserted into an area to be sutured is connected to a front end of the suture body 10, and a combining method and principle of the needle to the suture body 10, such as binding using a loop or a protrusion generally formed on the needle, bonding using a bonding material, compression using a tube formed at the tip of the needle, swaging, forming or wrapping, are easily understood by those skilled in the art and a detailed description thereof will thus be omitted.

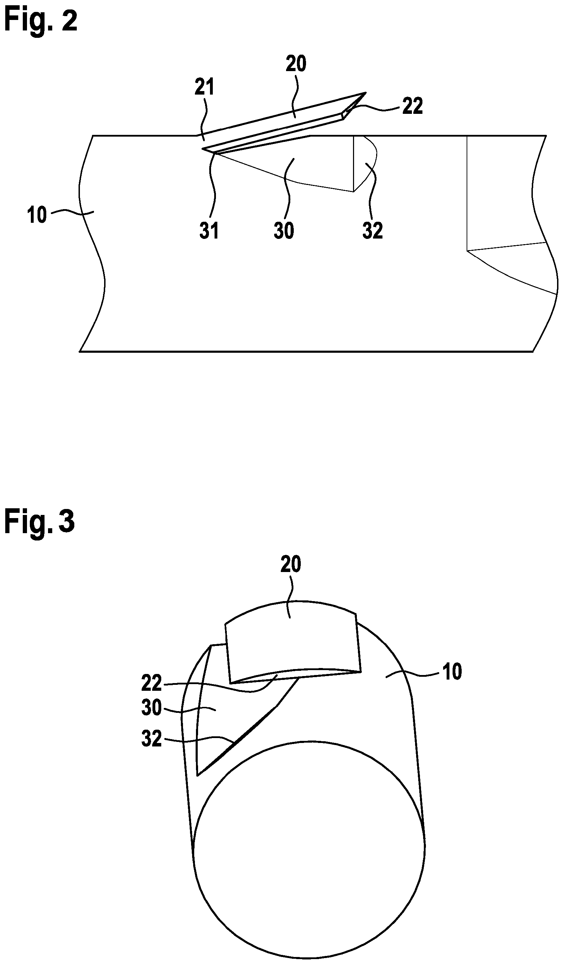

Particularly, in the adhesive barbed suture in accordance with the present invention, referring to FIGS. 2 to 7, the barb grooves 30 having the tail ends 31 and the front ends 32 are formed at regular intervals on the outer peripheral surface of the suture body 10 along a spiral path of the suture body, the barbs 20 having the tail ends 21 and the front ends 22 to have a shape corresponding to the barb grooves 30 having the tail ends 31 and the front ends 32 are formed at the same intervals as the barb grooves 30 in the axial direction of the suture body 10, and thereby, the adhesive force of the barbed suture is increased by a combination of stress by external force in the spirally inclined direction applied to the barb grooves 30 having the tail ends 31 and the front ends 32 and stress by external force in the axial direction applied to the barbs 20 having the tail ends 21 and the front ends 22.

That is, as shown in FIG. 1, Korean Patent Registration No. 10-1172829 discloses the barbed suture in which both barbs and cut grooves formed in a spiral direction on the outer peripheral surface of the barbed suture are aligned in the coaxial direction of a suture body, or are aligned at a spiral inclination angle, and thus, the barbs do not withstand load in one direction occurring in the longitudinal axial direction or the spiral direction of the barbed suture and may thus be torn.

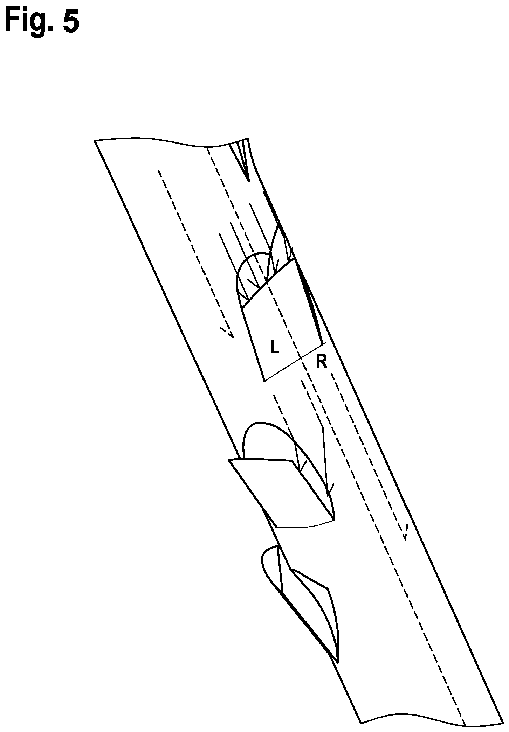

Accordingly, in the adhesive barbed suture in accordance with the present invention, referring to FIG. 5, the barb groove 30 is aligned in the spirally inclined direction so as to cope with stress by external force in the spirally inclined direction, the barb 20 is aligned in the axial direction so as to cope with stress by external force in the axial direction, and thus, resistance due to stress in two directions against load applied in the longitudinal axial direction of the suture occurs and thereby adhesiveness of the suture is increased.

Due to such a functional principle, when the suture including the barb grooves 30 and the barbs 20 is inserted into a human body, biological tissues are generally regenerated and engrafted into the barb grooves 30 within at least several days to one month through vital reaction and regeneration behavior, thereby enabling strong spiral-type fixation between the suture and surrounding tissues.

Further, the spiral-type fixation between the suture and tissues surrounding the suture due to the barb grooves 30 serves to increase physical friction with respect to sliding or movement of the suture in the tensile direction, and serves to apply torsional moment to the tissues surrounding the suture by external force in the tensile direction occurring due to movement of a region into which the suture is inserted or a sutured area.

Increase in resistance to movement in the tensile direction due to rotation of a slender object (for example, an object, such as a needle or a suture) which is inserted into biological tissue is widely known through various research on a "grasp" phenomenon (Helene M. Langevin, David L. Churchill, Junru Wu, Gary J. Badger, Jason A. Yandow, James R. Fox, and Martin H. Krag, Evidence of Connective Tissue Involvement in Acupuncture, The FASEB Journal, Published online April 10, 2002.).

Moreover, as the barbs 20 are aligned in the axial direction, resistance of the barbs to external force in the axial direction is applied perpendicular to the external force applied in the axial direction, the maximum resistive moment is applied, and thus resistance to sliding in the axial direction is maximized.

Thereby, in the adhesive barbed suture in accordance with the present invention, spiral resistance by the barb grooves 30 and axial resistance by the barbs 20 are simultaneously applied in two directions, and thus, adhesive force of the adhesive barbed suture is maximized.

Based on the above-described principles, the adhesive barbed suture in accordance with the present invention may maintain firm adhesive force in a corresponding treatment area without drawbacks of the conventional barbed sutures, i.e., sliding or movement of the suture in an inserted direction over time.

Particularly, this adhesive property serves to lower the possibility of serious side effects, such as rupture of a sutured area, to enable more safe suturing, to prevent the inserted suture from sliding in the opposite direction to lifting and to maintain fixation of lifted tissue, and corresponds to useful performance which is required in a surgical operation, such as tissue lift surgery.

Further, the barb grooves 30 and the barbs 20 are configured, as shown in FIGS. 2 and 3, such that the tail end 21 of the barb and the tail end 31 of the barb groove, which are in contact with the outer peripheral surface of the suture body 10, are integrally bonded to the suture body at the same position on the outer peripheral surface of the suture body, and the front end 22 of the barb 20 is spaced apart from the front end 32 of the barb groove 30 by being upwardly inclined at a certain angle on the outer peripheral surface of the suture body.

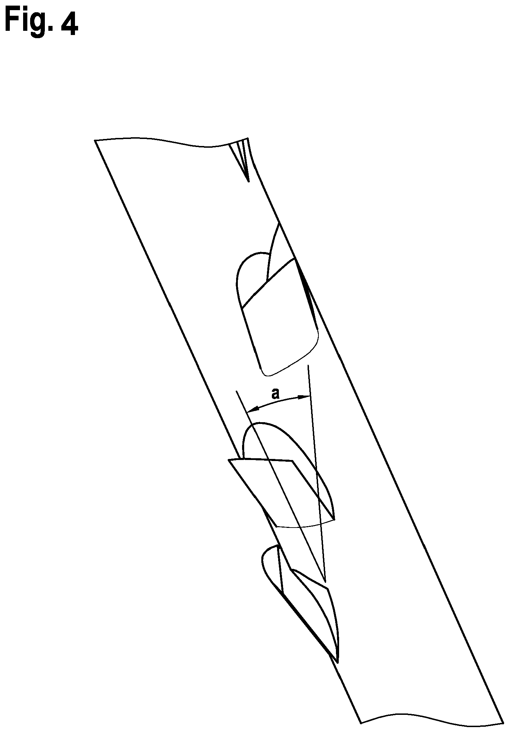

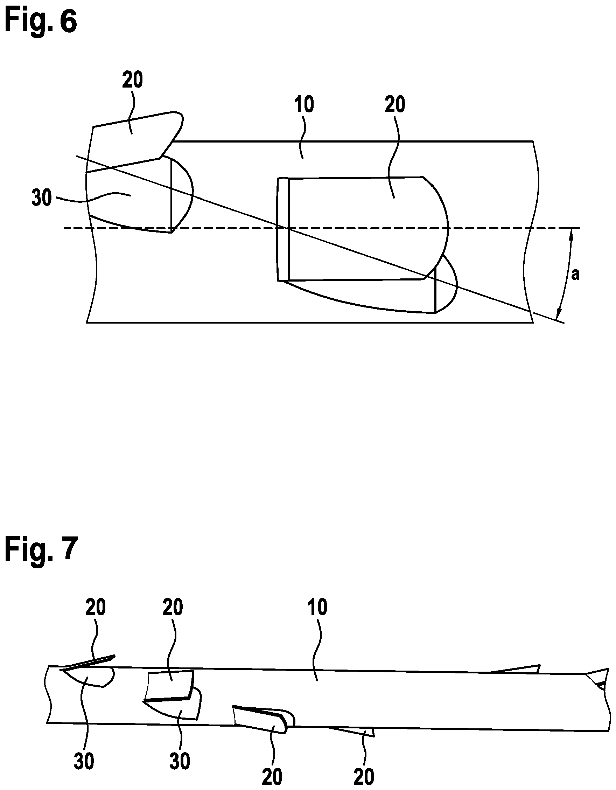

Also, as shown in FIGS. 4 and 6, the barbs 20 and the barb grooves 30 are formed at regular intervals along the spiral path of the suture body 10, the front end 22 of the barb 20 is aligned in the longitudinal direction toward the axial direction of the suture body, and the front end 32 of the barb groove 30 is aligned in the longitudinal direction toward the spiral direction of the suture body while having a spiral angle a with respect to the front end 22 of the barb 10.

Here, the spiral angle a of the front end of the barb groove with respect to the front end of the barb may be within the range of 3-80 degrees so as to maximize adhesive force, and, when the spiral angle a is less than 3 degrees, stress by external force in the spirally inclined direction is insufficient and, when the spiral angle a exceeds 80 degrees, the barbs and the barb grooves are formed over an excessively wide area and thus the suture body may be damaged.

When the barbs and the barb grooves are formed at the regular intervals along the spiral path of the suture body, the interval may be less than 3 times the diameter of the suture body, and, when the interval is 3 times or more the diameter of the suture body, the intervals between the barbs and between the barb grooves are excessively long, the number of the barbs and the barb grooves is small, and thus adhesive force of the barbed suture is decreased.

Further, the front ends of the barbs and the front ends of the barb grooves are formed with a regular thickness so as to have a designated angle. That is, referring to FIG. 2, the front ends 22 of the barbs are formed with a regular thickness so as to have a designated angle, the barb grooves 30 are formed so as to surround the outer peripheral surface of the suture body 10 in the longitudinal direction of the suture body 10 and to be spaced apart from one another at the regular intervals, the barb grooves 30 are indented in the inward direction from the overall outer diameter of the suture body 10 so as to have a regular depth and length, and the front ends 32 of the barb grooves 30 may be formed with a regular thickness so as to have a designated angle.

The adhesive barbed suture in accordance with the present invention may be produced so as to have a designated length through injection molding by injecting a liquid resin into a mold cavity having a shape of the suture body configured such that the barb grooves having the tail ends and the front ends and the barbs having the tail ends and the front ends to have a shape corresponding to the barb grooves having the tail ends and the front ends are formed at the regular intervals on the outer peripheral surface of the suture body, the tail end of the barb and the tail end of the barb groove, which are in contact with the outer peripheral surface of the suture body, are integrally bonded to the suture body at the same position on the outer peripheral surface of the suture body, the front end of the barb is spaced apart from the front end of the barb groove by being upwardly inclined at the certain angle on the outer peripheral surface of the suture body, the barbs and the barb grooves are formed at the regular intervals along the spiral path of the suture body, the front end of the barb is aligned in the longitudinal direction toward the axial direction of the suture body, and the front end of the barb groove is aligned in the longitudinal direction toward the spiral direction of the suture body while having the spiral angle with respect to the front end of the barb.

Further, the adhesive barbed suture in accordance with the present invention may be produced by forming the barbs having the tail ends and the front ends to have a shape corresponding to the barb grooves having the tail ends and the front ends by forming the barb grooves having the tail ends and the front ends at regular intervals on the outer peripheral surface of the suture body so that the tail end of the barb and the tail end of the barb groove, which are in contact with the outer peripheral surface of the suture body, are integrally bonded to the suture body at the same position on the outer peripheral surface of the suture body, the front end of the barb is spaced apart from the front end of the barb groove by being upwardly inclined at the certain angle on the outer peripheral surface of the suture body, through cutting using a blade cutting machine or a laser cutting machine, and then by twisting the suture body by axially rotating the suture body so that the barbs and the barb grooves are formed at the regular intervals along the spiral path of the suture body, the front end of the barb is aligned in the longitudinal direction toward the axial direction of the suture body, and the front end of the barb groove is aligned in the longitudinal direction toward the spiral direction of the suture body while having the spiral angle with respect to the front end of the barb.

In addition, the adhesive barbed suture in accordance with the present invention may be produced by forming the barb grooves having the tail ends and the front ends and the barbs having the tail ends and the front ends to have a shape corresponding to the barb grooves having the tail ends and the front ends at regular intervals on the outer peripheral surface of the suture body so that the tail end of the barb and the tail end of the barb groove, which are in contact with the outer peripheral surface of the suture body, are integrally bonded to the suture body at the same position on the outer peripheral surface of the suture body, the front end of the barb is spaced apart from the front end of the barb groove by being upwardly inclined at the certain angle on the outer peripheral surface of the suture body, through compression molding using a compression molding machine, and then by twisting the suture body by axially rotating the suture body so that the barbs and the barb grooves are formed at the regular intervals along the spiral path of the suture body, the front end of the barb is aligned in the longitudinal direction toward the axial direction of the suture body, and the front end of the barb groove is aligned in the longitudinal direction toward the spiral direction of the suture body while having the spiral angle with respect to the front end of the barb.

Here, in order to form the barb grooves 30 along the spiral path of the suture body and to more effectively form the barbs 20 in the axial direction of the suture body, a heat treatment process may be further performed. In more detail, in order to align the barbs 20 in the axial direction without distortion, the barbs may undergo the heat treatment process for minimizing distortion and thus be completely aligned in the axial direction.

That is, the heat treatment process is a method in which a polymer is heat-treated at a temperature around a glass transition temperature lower than a melting point of the polymer for a designated time so as to be deformed to a desired shape.

In this method, polymer chains may be rearranged around the glass transition temperature, and simultaneously, the polymer may be oriented by applying physical force thereto. Further, as a similar heat treatment method, there is a method in which a polymer is heat-treated at a high temperature above the glass transition temperature for a designated time so as to be deformed to a desired shape and is then cooled, and this method uses recrystallization of the polymer.

Through the above heat treatment, the barb grooves 30 may be formed along the spiral path of the suture body, and the barbs 20 may be oriented in the axial direction of the suture body.

Further, the adhesive barbed suture in accordance with the present invention may be produced by forming the barb grooves having the tail ends and the front ends and the barbs having the tail ends and the front ends to have a shape corresponding to the barb grooves having the tail ends and the front ends at regular intervals on the outer peripheral surface of the suture body so that the tail end of the barb and the tail end of the barb groove, which are in contact with the outer peripheral surface of the suture body, are integrally bonded to the suture body at the same position on the outer peripheral surface of the suture body, the front end of the barb is spaced apart from the front end of the barb groove by being upwardly inclined at the certain angle on the outer peripheral surface of the suture body, through compression molding using a compression molding machine, and then by performing compression molding while axially rotating the suture body so that the barbs and the barb grooves are formed at the regular intervals along the spiral path of the suture body, the front end of the barb is aligned in the longitudinal direction toward the axial direction of the suture body, and the front end of the barb groove is aligned in the longitudinal direction toward the spiral direction of the suture body while having the spiral angle with respect to the front end of the barb, without a separate heat treatment process.

Further, in an adhesive barbed suture in accordance with another embodiment of the present invention, in which barbs and barb grooves are formed on the outer peripheral surface of a suture body, a tail end of the barb and a tail end of the barb groove, which are in contact with the outer peripheral surface of the suture body, are integrally bonded to the suture body at the same position on the outer peripheral surface of the suture body, a front end of the barb is spaced apart from a front end of the barb groove by being upwardly inclined at a certain angle on the outer peripheral surface of the suture body, the barbs and the barb grooves are formed at regular intervals along a spiral path of the suture body, and the front end of the barb is aligned in the longitudinal direction toward the axial direction of the suture body and the front end of the barb groove is aligned in the longitudinal direction toward the spiral direction of the suture body while having a spiral angle with respect to the front end of the barb so that the adhesive force of the barbed suture is increased by means of combined biaxial stress in which stress by external force in the axial direction applied to the front end of the barb and stress by external force in the spiral direction applied to the front end of the barb groove are simultaneously applied, one protruding barb or two or more protruding barbs, selected from the group consisting of a wedge-type protruding barb, a right-angled triangular protruding barb, a wedge-type conical protruding barb, a right-angled conical protruding barb and a knot-type protruding barb, may be formed on the outer peripheral surface of the suture body within the longitudinal interval between the barb and the barb.

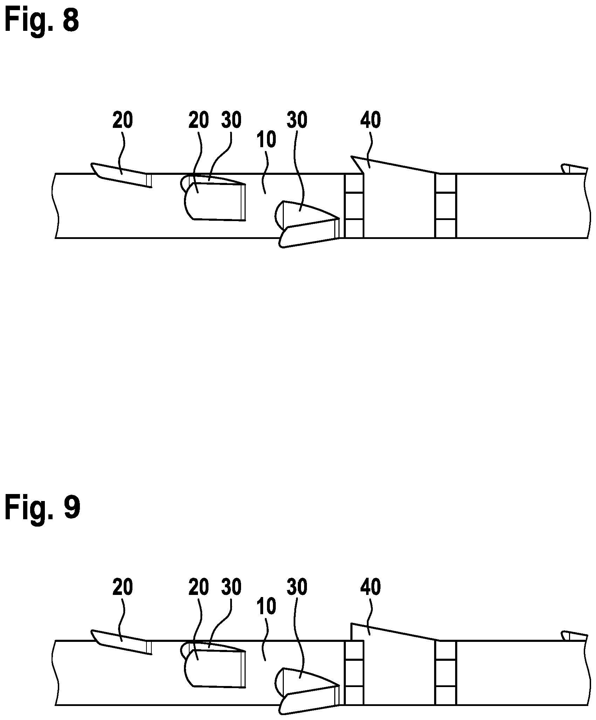

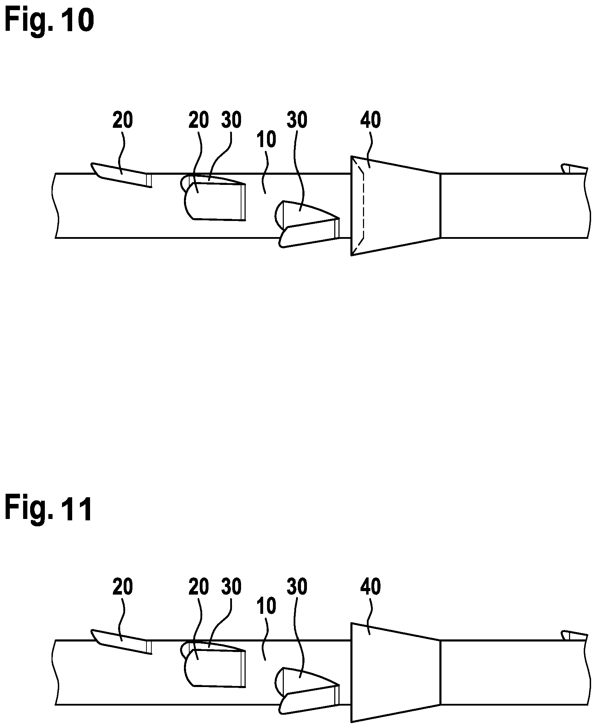

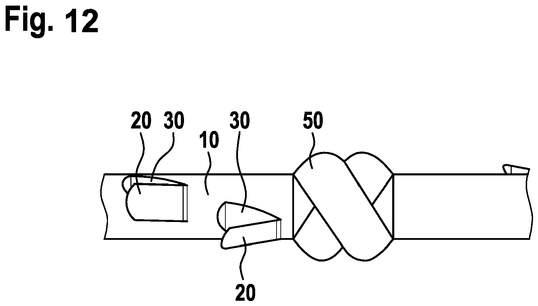

In more detail, a wedge-type protruding barb 40 may be formed on the outer peripheral surface of the suture body within the longitudinal interval between the barb and the barb, as shown in FIG. 8, a right-angled triangular protruding barb 40 may be formed on the outer peripheral surface of the suture body within the longitudinal interval between the barb and the barb, as shown in FIG. 9, a wedge-type conical protruding barb 40 may be formed on the outer peripheral surface of the suture body within the longitudinal interval between the barb and the barb, as shown in FIG. 10, a right-angled conical protruding barb 40 may be formed on the outer peripheral surface of the suture body within the longitudinal interval between the barb and the barb, as shown in FIG. 11, and a knot-type protruding barb 50 may be formed on the outer peripheral surface of the suture body within the longitudinal interval between the barb and the barb, as shown in FIG. 12.

Since the protruding barbs are oriented in the axial direction so that the barbs of the adhesive barbed suture in accordance with the present invention cope with stress by external force in the axial direction, in addition to increase in adhesive force of the suture caused by resistance occurring due to stress in two directions against load applied in the longitudinal axial direction, the protruding barbs further increase the adhesive force of the suture.

Further, of course, these protruding barbs may be formed through injection molding, compression molding, joined molding or knot forming.

Although the preferred embodiments of the present invention have been disclosed for illustrative purposes, those skilled in the art will appreciate that various modifications, additions and substitutions are possible, without departing from the scope and spirit of the invention as disclosed in the accompanying claims. Thus, the drawings disclosed in the present invention do not limit the technical idea of the invention but merely describe the invention, and the scope of the technical idea of the invention is not limited by the drawings. Thus, it is intended that the present invention cover the modifications and variations of the invention provided they come within the scope of the appended claims and their equivalents.

### [Industrial Applicability]

As is apparent from the above description, in an adhesive barbed suture and a method for producing the same in accordance with the present invention, a tail end of a barb and a tail end of a barb groove, which are in contact with the outer peripheral surface of a suture body, are integrally bonded to the suture body at the same position on the outer peripheral surface of the suture body, a front end of the barb is spaced apart from a front end of the barb groove by being upwardly inclined at a certain angle on the outer peripheral surface of the suture body, the barbs and the barb grooves are formed at regular intervals along a spiral path of the suture body, and the front end of the barb is aligned in the longitudinal direction toward the axial direction of the suture body and the front end of the barb groove is aligned in the longitudinal direction toward the spiral direction of the suture body while having a spiral angle with respect to the front end of the barb so that the adhesive force of the barbed suture is increased by means of combined biaxial stress in which stress by external force in the axial direction applied to the front end of the barb and stress by external force in the spiral direction applied to the front end of the barb groove are simultaneously applied, and thus knots are not necessary when using the suture, and the suture may be rapidly and firmly fixed by maximizing fixing force between tissues and surrounding tissues, and the suture during suturing, and therefore, the adhesive barbed suture and the method for producing the same are industrially applicable to a medical instrument field.

## Claims

1. An adhesive barbed suture having barbs and barb grooves formed on an outer peripheral surface of a suture body,
wherein a tail end of the barb and a tail end of the barb groove, which are in contact with the outer peripheral surface of the suture body, are integrally bonded to the suture body at the same position on the outer peripheral surface of the suture body, a front end of the barb is spaced apart from a front end of the barb groove by being upwardly inclined at a certain angle on the outer peripheral surface of the suture body, the barbs and the barb grooves are formed at regular intervals along a spiral path of the suture body, and the front end of the barb is aligned in a longitudinal direction toward an axial direction of the suture body and the front end of the barb groove is aligned in the longitudinal direction toward a spiral direction of the suture body while having a spiral angle with respect to the front end of the barb so that adhesive force of the barbed suture is increased by means of combined biaxial stress in which stress by external force in the axial direction applied to the front end of the barb and stress by external force in the spiral direction applied to the front end of the barb groove are simultaneously applied.

2. The adhesive barbed suture according to claim 1, wherein the spiral angle of the front end of the barb groove with respect to the front end of the barb is within a range of 3-80 degrees.

3. The adhesive barbed suture according to claim 1, wherein, when the barbs and the barb grooves are formed at the regular intervals along the spiral path of the suture body, the interval is less than 3 times a diameter of the suture body.

4. The adhesive barbed suture according to claim 1, wherein the front end of the barb and the front end of the barb groove are formed with a regular thickness so as to have a designated angle.

5. The adhesive barbed suture according to claim 1, wherein one protruding barb or two or more protruding barbs, selected from the group consisting of a wedge-type protruding barb, a right-angled triangular protruding barb, a wedge-type conical protruding barb, a right-angled conical protruding barb and a knot-type protruding barb, are formed on the outer peripheral surface of the suture body within the longitudinal interval between the barb and the barb.

6. A method for producing the adhesive barbed suture according to any one of claims 1 to 4, wherein the adhesive barbed suture is produced so as to have a designated length through injection molding by injecting a liquid resin into a mold cavity having a shape of the suture body configured such that the barb grooves having the tail ends and the front ends and the barbs having the tail ends and the front ends to have a shape corresponding to the barb grooves having the tail ends and the front ends are formed at the regular intervals on the outer peripheral surface of the suture body, the tail end of the barb and the tail end of the barb groove, which are in contact with the outer peripheral surface of the suture body, are integrally bonded to the suture body at the same position on the outer peripheral surface of the suture body, the front end of the barb is spaced apart from the front end of the barb groove by being upwardly inclined at the certain angle on the outer peripheral surface of the suture body, the barbs and the barb grooves are formed at the regular intervals along the spiral path of the suture body, the front end of the barb is aligned in the longitudinal direction toward the axial direction of the suture body, and the front end of the barb groove is aligned in the longitudinal direction toward the spiral direction of the suture body while having the spiral angle with respect to the front end of the barb.

7. A method for producing the adhesive barbed suture according to any one of claims 1 to 4, wherein the adhesive barbed suture is produced by forming the barbs having the tail ends and the front ends to have a shape corresponding to the barb grooves having the tail ends and the front ends by forming the barb grooves having the tail ends and the front ends at the regular intervals on the outer peripheral surface of the suture body so that the tail end of the barb and the tail end of the barb groove, which are in contact with the outer peripheral surface of the suture body, are integrally bonded to the suture body at the same position on the outer peripheral surface of the suture body, the front end of the barb is spaced apart from the front end of the barb groove by being upwardly inclined at the certain angle on the outer peripheral surface of the suture body, through cutting using a blade cutting machine or a laser cutting machine, and then by twisting the suture body by axially rotating the suture body so that the barbs and the barb grooves are formed at the regular intervals along the spiral path of the suture body, the front end of the barb is aligned in the longitudinal direction toward the axial direction of the suture body, and the front end of the barb groove is aligned in the longitudinal direction toward the spiral direction of the suture body while having the spiral angle with respect to the front end of the barb.

8. A method for producing the adhesive barbed suture according to any one of claims 1 to 4, wherein the adhesive barbed suture is produced by forming the barb grooves having the tail ends and the front ends and the barbs having the tail ends and the front ends to have a shape corresponding to the barb grooves having the tail ends and the front ends at the regular intervals on the outer peripheral surface of the suture body so that the tail end of the barb and the tail end of the barb groove, which are in contact with the outer peripheral surface of the suture body, are integrally bonded to the suture body at the same position on the outer peripheral surface of the suture body, the front end of the barb is spaced apart from the front end of the barb groove by being upwardly inclined at the certain angle on the outer peripheral surface of the suture body, through compression molding using a compression molding machine, and then by twisting the suture body by axially rotating the suture body so that the barbs and the barb grooves are formed at the regular intervals along the spiral path of the suture body, the front end of the barb is aligned in the longitudinal direction toward the axial direction of the suture body, and the front end of the barb groove is aligned in the longitudinal direction toward the spiral direction of the suture body while having the spiral angle with respect to the front end of the barb.

9. A method for producing the adhesive barbed suture according to any one of claims 1 to 4, wherein the adhesive barbed suture is produced by forming the barb grooves having the tail ends and the front ends and the barbs having the tail ends and the front ends to have a shape corresponding to the barb grooves having the tail ends and the front ends at the regular intervals on the outer peripheral surface of the suture body so that the tail end of the barb and the tail end of the barb groove, which are in contact with the outer peripheral surface of the suture body, are integrally bonded to the suture body at the same position on the outer peripheral surface of the suture body, the front end of the barb is spaced apart from the front end of the barb groove by being upwardly inclined at the certain angle on the outer peripheral surface of the suture body, through compression molding using a compression molding machine, and then by performing compression molding while rotating the suture body so that the barbs and the barb grooves are formed at the regular intervals along the spiral path of the suture body, the front end of the barb is aligned in the longitudinal direction toward the axial direction of the suture body, and the front end of the barb groove is aligned in the longitudinal direction toward the spiral direction of the suture body while having the spiral angle with respect to the front end of the barb.
